(19) Europäisches Patentamt

European Patent Office

Office européen des brevets

(11) Veröffentlichungsnummer: **0 285 871 B1**

(12) **EUROPÄISCHE PATENTSCHRIFT**

(45) Veröffentlichungstag der Patentschrift: **10.06.92**

(21) Anmeldenummer: **88104165.1**

(22) Anmeldetag: **16.03.88**

(51) Int. Cl.⁵: **A61K 9/16**

(54) **Bindemittelfreies Granulat mit verzögerter Wirkstoffabgabe.**

(30) Priorität: **10.04.87 DE 3712095**

(43) Veröffentlichungstag der Anmeldung:
**12.10.88 Patentblatt 88/41**

(45) Bekanntmachung des Hinweises auf die
Patenterteilung:
**10.06.92 Patentblatt 92/24**

(84) Benannte Vertragsstaaten:
**AT BE CH DE ES FR GB GR IT LI LU NL SE**

(56) Entgegenhaltungen:
**EP-A- 0 108 882**
**EP-A- 0 133 988**
**EP-A- 0 172 422**
**FR-A- 2 400 950**

(73) Patentinhaber: **PCD Polymere Gesellschaft
m.b.H.
Danubiastrasse 21-25
A-2323 Schwechat-Mannswörth(AT)**

(72) Erfinder: **Korsatko, Werner, Dr.
Kärntnerstrasse 432
A-8054 Graz(AT)**
Erfinder: **Korsatko-Wabnegg, Brigitta, Dr.
Kärntnerstrasse 432
A-8054 Graz(AT)**

(74) Vertreter: **Kunz, Ekkehard, Dr.
Chemie Holding AG Patentwesen St. Peter-
Strasse 25
A-4021 Linz(AT)**

**Beschreibung**

Die Erfindung betrifft ein bindemittelfreies Granulat mit verzögerter Wirkstoffabgabe.

Es ist bekannt, daß es für bestimmte Wirkstoffe von Vorteil ist, Arzneiformen herzustellen, die den Wirkstoff über einen langanhaltenden Zeitraum kontinuierlich abgeben. Für eine Anzahl pharmakologisch wirksamer Substanzen sind daher galenische Darreichungsformen mit einer verzögerten Wirkstoffabgabe für die orale, parenterale und topische Applikation entwickelt worden.

Eine solche Retardwirkung kann man beispielsweise durch Überziehen von Pulvern und Granulaten mit Acrylharzlacken erreichen. Diese Überzüge und üblicherweise auch die zu überziehenden Arzneiformen enthalten zusätzliche Hilfs- und Füllstoffe, vor allem sind jedoch Acrylharzlacke körperfremde Substanzen und biologisch nicht abbaubar.

Nach US-PS 3,773.919 können bestimmte, biologisch abbaubare Polymere, nämlich Polyglykolsäure, Polymilchsäure und Copolymerisate von Milch- und Glykolsäure zur Herstellung von Retardformen verwendet werden. Die Herstellung dieser Retardformen kann dabei A) durch Beschichten wie Sprühtrocknen, Fließbettbeschichten oder Mikroeinkapseln, B) durch Einbetten ("embedding") oder C) durch enges Vermischen, wie Lösen von Wirkstoff und Polymer in einem Lösungsmittel und Verdampfen des Lösungsmittels erfolgen. In den Beispielen ist allerdings nur die Herstellung von Retardformen durch "embedding" beschrieben, daß heißt, das Polylactid wird geschmolzen, der Wirkstoff eingebracht und in der Schmelze suspendiert, die Schmelze erstarren gelassen und in kleine Partikel zerrieben. Bei dieser Methode besteht jedoch die Gefahr, daß der Wirkstoff thermisch zersetzt wird. Weiters zeigte sich beim Nacharbeiten der dort im allgemeinen Teil unter A) beschriebenen Methode des Wirbelschichtgranulierens, daß sich die dort angegebenen Polylactide für Wirbelschichtgranulierung nicht eignen, da die Polymerlösung beim Versprühen in unterschiedlichen Konzentrationen und unter Verwendung unterschiedlicher Lösungsmittel immer Fäden zog und kein gleichmäßiger Überzug des Wirkstoffes erhalten werden konnte.

Aus EP-A-0 108 882 ist bekannt, Poly-D(-)-3-hydroxybuttersäure, im folgenden Poly-HB genannt, als polymeres Trägermaterial zu verwenden, wobei die Poly-HB direkt mit dem Wirkstoff vermischt und zu Tabletten verpreßt wird, in denen der Wirkstoff gleichmäßig in einem Poly-HB-Gerüst verteilt ist. Zur Erzielung einer Retardwirkung von mehreren Stunden sind jedoch mindestens 20 Gew.% Poly-HB notwendig. Dies ist insbesondere für Arzneimittel, die hochdosiert werden müssen und deshalb große Wirkstoffmengen enthalten, von Nachteil. EP-A-0 133 988 betrifft ein Implantat, mit Poly-HB als Träger in relativ hohe Mengen; die Wirkstoffteilchen in diesem Implantat sind nicht mit einer Poly-HB Schicht überzogen. Darüber hinaus gibt es Problemarzneistoffe, die ungünstige osmotische Eigenschaften haben, sodaß erst bei über 50 Gew.% Poly-HB Anteil eine stabile Matrix aufgebaut und eine gute Retardwirkung erzielt werden kann. Tabletten, die z.B. 200 mg Wirkstoff enthalten, müssen demnach bei dieser Methode mit mindestens 200 mg Poly-HB vermischt werden. Die dabei enstehenden Tabletten von über 400 mg Gewicht sind so groß, daß ihre Einnahme für den Patienten schon unangenehm und sogar beschwerlich ist. Es stellte sich daher die Aufgabe, Retardtabletten herzustellen, die nur geringe Mengen an biologisch abbaubarem, polymeren Material bei gleichzeitig hoher Retardwirkung enthalten, wodurch eine große Wirkstoffmenge in einer relativ kleinen Retardtablette enthalten ist.

Es wurde nun gefunden, daß es möglich ist, ausgehend von einem Wirkstoff oder dessen Granulat mit Hilfe von Poly-HB ein bindemittelfreies Granulat herzustellen, das nur mit einer geringen Menge an Poly-HB überzogen ist, das gleichzeitig gute Retardwirkung aufweist und ohne Zusatz von Hilfsstoffen zu Tabletten verpreßbar ist.

Gegenstand der Erfindung ist demnach ein bindemittelfreies Granulat zur oralen und parenteralen Applikation mit verzögerter Wirkstofffreisetzung bestehend aus einem pharmazeutischen Wirkstoff oder dessen Granulat und Poly-D(-)-3-hydroxybuttersäure, dadurch gekennzeichnet, daß das Wirkstoffgranulat mit einer Menge von 1 bis 20 Gew.% Poly-D(-)-3-hydroxybuttersäure bezogen auf das Gesamtgewicht, überzogen ist.

Das erfindungsgemäße Granulat besteht nur aus dem Wirkstoff oder dessen Granulat, welche mit einem bestimmten Anteil an Poly-HB überzogen sind. Es besitzt gute Fließeigenschaften und kann überraschenderweise ohne zusätzliche Hilfs- oder Füllstoffe problemlos zu Tabletten verpreßt werden.

Man kann prinzipiell jeden Wirkstoff oder dessen Granulat einsetzen und erhält so umfassende Anwendungsmöglichkeiten. Ein Beispiel hiefür ist der Wirkstoff 7-Hydroxyethyltheophyllin. Eine besondere Bedeutung kommt der Erfindung bei Tabletten mit großen Wirkstoffmengen zu, da sich die Tablettengröße durch den reduzierten Poly-HB-Gehalt erheblich verringern läßt. Beispiele für solche Wirkstoffe sind Celiprololhydrochlorid, Hexobendindihydrochlorid, Ibuprofen, Diclofenac-Na, etc.

Die erzielte Retardwirkung ist einerseits von den physikalischen Eigenschaften des verwendeten Wirkstoffes abhängig, andererseits von der Dicke des Überzuges mit Poly-HB sowie vom Molekulargewicht der verwendeten Poly-HB. Je dicker der Überzug und/oder je höher das Molekulargewicht der verwendeten Poly-HB sind, desto langsamer wird der Wirkstoff freigesetzt, sodaß für jeden Wirkstoff 2 Parameter zur Erreichung einer gewünschten Retardzeit zur Verfügung stehen. Der Überzug an Poly-HB beträgt 1 bis 20 Gew.% bezogen auf das Gesamtgewicht des Granulates, bevorzugt 1 bis 15 Gew.%. Besonders bevorzugt ist der Bereich von 3 bis 10 Gew.%.

Es ist ein wesentliches Kennzeichen der Erfindung, daß viel Wirkstoff mit wenig Poly-HB umhüllt werden kann, wobei trotzdem gute Retardeigenschaften erreicht werden.

Das Abbauprodukt der Poly-HB, die D(-)-3-Hydroxybuttersäure ist eine körpereigene Substanz und kann daher keine Nachteile im Metabolismus haben.

Die verwendete Poly-HB stellt man beispielsweise nach EP-A-0 149 774 biotechnologisch durch aerobe Kultivierung eines Mikroorganismus der Gattung Alcaligenes her. Zur Herstellung des erfindungsgemäßen Granulates wird üblicherweise eine Poly-HB eines Molekulargewichtes von etwa 50.000 bis etwa 800.000 verwendet, wobei der Bereich von 100.000 bis 400.000 besonders bevorzugt ist.

Gegenstand der Erfindung ist auch ein Verfahren zur Herstellung eines bindemittelfreien Granulates, das dadurch gekennzeichnet ist, daß ein pharmazeutischer Wirkstoff oder dessen Granulat in einem Granulierungsverfahren mit einer Lösung von Poly-D(-)-3-hydroxybuttersäure besprüht und das Lösungsmittel verdampft wird.

In diesem Verfahren wird Poly-HB in einem für sie geeigneten Lösungsmittel wie beispielsweise Methylenchlorid oder Chloroform gelöst. Die Menge des Lösungsmittels ist abhängig von der Art des Lösungsmittels, vom Molekulargewicht der verwendeten Poly-HB und von der Sprühtemperatur. Die Sprühtemperatur beträgt etwa zwischen 35 und 50 °C. Der Wirkstoff wird als Pulver oder als Granulat eingesetzt und anschließend durch ein beliebiges aufbauendes Granulationsverfahren mit einer Lösung von Poly-HB besprüht, worauf nach Verdampfen des Lösungsmittels das erfindungsgemäße Granulat erhalten wird.

Besonders vorteilhaft ist der Einsatz der Wirbelschichtgranulierung, denn bei diesem Verfahren werden alle Verfahrensschritte in demselben Behälter ausgeführt. Der Wirkstoff oder dessen Granulat wird vorgelegt und mit einer Poly-HB-Lösung besprüht. Durch Zufuhr von Luft oder Inertgas wird eine Wirbelschicht aufgebaut und erhalten, wobei der Wirkstoff oder dessen Granulat unter Abdampfen des Lösungsmittels mit einer dünnen Schicht von Poly-HB überzogen wird.

Es entsteht ein poröses Granulat von sphärischer Form, das ohne Zusatz von Bindemitteln oder sonstigen Hilfsstoffen zu Komprimaten beliebiger Form verpreßt werden kann.

Beispiel 1:

100 g Celiprololhydrochlorid-Granulat wurde im Wirbelschichtgranulierungsbehälter vorgelegt und mit 300 ml einer Lösung von 6 g Poly-D(-)-3-hydroxybuttersäure in Chloroform (2%ige Lösung) vom Molekulargewicht 142.935 mit einem Sprühdruck von 1 bar und bei einer Temperatur von 40 °C besprüht. Durch Einblasen von Luft wurde dabei eine Wirbelschicht aufgebaut und das Lösungmittel abgedampft, wobei das Granulat mit einem dünnen Überzug von Poly-HB versehen wurde.

Der Gehalt des Wirbelschichtgranulates (WSG) an Poly-HB betrug 5 bis 6 % bezogen auf das Gesamtgewicht. Aus dem entstandenen Granulat wurden mit einem Druck von 2,5 t, das entspricht 245,3 N/mm$^2$, Tabletten hergestellt.

| | |
|---|---|
| Schüttvolumen des WSG: | 3,8 ml/g |
| Rüttelvolumen des WSG: | 3,09 ml/g |
| Tablettengröße: | d = 9,2 mm h = 4,2 mm |
| Durchschnittsgewicht der Tabletten: | 218,8 mg ± 5,3 mg (± 2,4 %) |
| Bruchfestigkeit: | 19,61 kp |

In analoger Weise wurden auch Granulat und Tabletten für die Beispiele 2 bis 9 hergestellt.

EP 0 285 871 B1

Tabelle I

| Beispiel | Wirkstoff | Molekulargewicht Poly-HB | %Poly- HB | Lösungs- mittel | Sprühtem- peratur °C |
|---|---|---|---|---|---|
| 1 | Celiprolol- hydrochlorid | 142.935 | 5 - 6 | CHCl₃ | 40 |
| 2 | " | 142.935 | 4 - 5 | " | 40 |
| 3 | " | 278.726 | 2 - 3 | " | 40 |
| 4 | " | 278.726 | 3 - 4 | " | 40 |
| 5 | " | 604.691 | 1 -2 | " | 40 |
| 6 | 7-Hydroxy- ethyltheophyllin | 604.691 | 1 | CHCl₃ | 40 |
| 7 | Hexobendin- dihydrochlorid | 604.691 | 1 | " | 40 |
| 8 | Ibuprofen | 604.691 | 1 | " | 40 |
| 9 | Diclofenac-Na | 604.691 | 1 | " | 40 |

In vitro Wirkstofffreigabe

Zur Messung der Retardeigenschaften der Celiprololhydrochlorid-Tabletten (Beispiele 1 bis 5) wurden die Tabletten in 100 ml 0,9%iger Natriumchloridlösung bei 37 °C in verschlossenen Braunglasflaschen geschüttelt und in Abständen von 30 bzw. 60 Minuten auf die freigesetzte Wirkstoffmenge untersucht. Auch die Half-change Methode, bei der der pH-Wert kontinuierlich innerhalb von 8 Stunden von pH 1,3 auf pH 7,3 erhöht wird, wobei in Abständen wie oben die Wirkstofffreisetzung gemessen wird, wurde zur Überprüfung angewandt und brachte gleiche Ergebnisse. Die quantitative analytische Erfassung von Celiprolol erfolgte spektralphotometrisch in geeigneter Verdünnung bei 324 nm. Die Retardeigenschaften der Tabletten mit den anderen Wirkstoffen (Beispiele 6 bis 9) wurden nach der Half-change Methode bestimmt. Die quantitative, analytische Erfassung erfolgte spektralphotometrisch (7-Hydroxyethyltheophyllin: 273 nm, Hexobendindihydrochlorid: 266 nm, Ibuprofen: 264 nm, Diclofenac-Na: 275 nm)

4

Tabelle II

FREIGESETZTE WIRKSTOFFMENGE in Gew.% bezogen auf den Gesamtgehalt

| Zeit (h) | 0,5 | 1 | 2 | 3 | 4 | 5 | 6 | 7 | 8 | 24 |
|---|---|---|---|---|---|---|---|---|---|---|
| Beispiel 1 | 17,46 | 26,33 | 40,62 | 52,29 | 58,81 | 64,87 | 70,26 | 77,36 | 80,49 | 95,53 |
| 2 | 26,42 | 42,72 | 63,05 | 76,74 | 86,06 | 92,15 | 94,73 | - | - | 98,77 |
| 3 | 22,08 | 34,96 | 55,66 | 68,94 | 76,69 | 84,19 | 88,62 | 91,76 | 92,68 | 98,38 |
| 4 | 17,91 | 27,60 | 42,26 | 54,37 | 61,41 | 71,36 | 75,82 | 81,39 | 86,52 | 99,99 |
| 5 | 28,06 | 47,08 | 69,44 | 83,82 | 89,88 | 95,61 | 95,88 | - | - | 97,31 |
| 6 | - | 24,61 | 41,05 | 56,80 | 61,77 | 69,43 | 75,43 | - | 85,31 | - |
| 7 | - | 14,70 | 27,21 | 41,34 | 48,88 | 58,72 | 67,40 | - | 77,73 | - |
| 8 | - | 1,11 | 1,64 | 5,49 | 8,82 | 13,14 | 16,89 | - | 24,08 | - |
| 9 | - | - | - | 5,50 | 9,78 | 13,71 | 29,03 | - | 42,62 | - |

Patentansprüche
Patentansprüche für folgende Vertragsstaaten : AT, BE, CH, DE, FR, GB, IT, LI, LU, NL, SE

1. Bindemittelfreies Granulat zur oralen und parenteralen Applikation mit verzögerter Wirkstofffreisetzung bestehend aus einem pharmazeutischen Wirkstoff oder dessen Granulat und Poly-D(-)-3-hydroxybuttersäure, dadurch gekennzeichnet, daß der Wirkstoff oder dessen Granulat mit einer Menge von 1 bis 20 Gew.% Poly-D(-)-3-hydroxybuttersäure bezogen auf das Gesamtgewicht, überzogen ist.

2. Bindemittelfreies Granulat nach Anspruch 1, dadurch gekennzeichnet, daß der Wirkstoff oder dessen Granulat mit einer Menge von 1 bis 15 % Poly-D(-)-3-hydroxybuttersäure bezogen auf das Gesamtgewicht, überzogen ist.

3. Bindemittelfreies Granulat nach Anspruch 2, dadurch gekennzeichnet, daß der Wirkstoff oder dessen Granulat mit einer Menge von 3 bis 10 % Poly-D(-)-3-hydroxybuttersäure bezogen auf das Gesamtgewicht, überzogen ist.

4. Bindemittelfreies Granulat nach einem der Ansprüche 1 bis 3, dadurch gekennzeichnet, daß das Molgewicht der eingesetzten Poly-D(-)-3-hydroxybuttersäure zwischen 50.000 und 800.000 liegt.

5. Bindemittelfreies Granulat nach einem der Ansprüche 1 bis 4, dadurch gekennzeichnet, daß das Molgewicht der verwendeten Poly-D(-)-3-hydroxybuttersäure zwischen 100.000 und 400.000 liegt.

6. Bindemittelfreies Granulat nach einem der Ansprüche 1 bis 5, dadurch gekennzeichnet, daß der Wirkstoff Celiprololhydrochlorid ist.

7. Bindemittelfreies Granulat nach einem der Ansprüche 1 bis 6, dadurch gekennzeichnet, daß es durch eine Wirbelschichtgranulierung hergestellt wurde.

8. Verfahren zur Herstellung eines bindemittelfreien Granulates zur oralen oder parenteralen Applikation mit verzögerter Wirkstofffreisetzung bestehend aus Poly-D(-)-3-hydroxybuttersäure und einem pharmazeutischen Wirkstoff, dadurch gekennzeichnet, daß der pharmazeutische Wirkstoff oder dessen Granulat in einem aufbauenden Granulationsverfahren mit einer Lösung von Poly-D(-)-3-hydroxy-buttersäure besprüht und das Lösungsmittel verdampft wird, wobei der Wirkstoff oder dessen Granulat mit einer Menge von 1 bis 20 Gew.% Poly-D(-)-3-hydroxybuttersäure bezogen auf das Gesamtgewicht überzogen wird.

9. Verfahren nach Anspruch 8, dadurch gekennzeichnet, daß das aufbauende Granulationsverfahren ein Wirbelschichtgranulierungsverfahren ist.

10. Verfahren nach einem der Ansprüche 7 bis 9, dadurch gekennzeichnet, daß das bindemittelfreie Granulat zu einer Arzneiform verpreßt wird.

**Patentansprüche für folgende Vertragsstaaten : ES, GR**

1. Verfahren zur Herstellung eines bindemittelfreien Granulates zur oralen oder parenteralen Applikation mit verzögerter Wirkstofffreisetzung bestehend aus Poly-D(-)-3-hydroxybuttersäure und einem pharmazeutischen Wirkstoff, dadurch gekennzeichnet, daß der pharmazeutische Wirkstoff oder dessen Granulat in einem aufbauenden Granulationsverfahren mit einer Lösung von Poly-D(-)-3-hydroxybuttersäure besprüht und das Lösungsmittel verdampft wird, wobei der Wirkstoff oder dessen Granulat mit einer Menge von 1 bis 20 Gew.% Poly-D(-)-3-hydroxybuttersäure bezogen auf das Gesamtgewicht überzogen wird.

2. Verfahren nach Anspruch 1, wobei der Wirkstoff oder dessen Granulat mit einer Menge von 1 bis 15 % Poly-D(-)-3-hydroxybuttersäure bezogen auf das Gesamtgewicht überzogen ist.

3. Verfahren nach Anspruch 2, dadurch gekennzeichnet, daß der Wirkstoff oder dessen Granulat mit einer Menge von 3 bis 10 % Poly-D(-)-3-hydroxybuttersäure bezogen auf das Gesamtgewicht, überzogen ist.

4. Verfahren nach einem der Ansprüche 1 bis 3, dadurch gekennzeichnet, daß das Molgewicht der eingesetzten Poly-D(-)-3-hydroxybuttersäure zwischen 50.000 und 800.000 liegt.

5. Verfahren nach einem der Ansprüche 1 bis 4, dadurch gekennzeichnet, daß das Molgewicht der verwendeten Poly-D(-)-3-hydroxybuttersäure zwischen 100.000 und 400.000 liegt.

6. Verfahren nach einem der Ansprüche 1 bis 5, dadurch gekennzeichnet, daß der Wirkstoff Celiprololhydrochlorid ist.

7. Verfahren nach einem der Ansprüche 1 bis 6, dadurch gekennzeichnet, daß das aufbauende Granulationsverfahren ein Wirbelschichtgranulierungsverfahren ist.

8. Verfahren nach einem der Ansprüche 1 bis 7, dadurch gekennzeichnet, daß das bindemittelfreie Granulat zu einer Arzneiform verpreßt wird.

**Claims**
**Claims for the following Contracting States : AT, BE, CH, DE, FR, GB, IT, LI, LU, NL, SE**

1. Binder-free granules for oral and parenteral administration with delayed release of the active compound consisting of a pharmaceutical active compound or granules thereof and poly-D(-)-3-hydroxybutyric acid, characterised in that the active compound or granules thereof is/are coated with an amount of 1 to 20% by weight of poly-D(-)-3-hydroxybutyric acid, based on the total weight.

2. Binder-free granules according to Claim 1, characterised in that the active compound or granules thereof is/are coated with an amount of 1 to 15% of poly-D(-)-3-hydroxybutyric acid, based on the total weight.

3. Binder-free granules according to Claim 2, characterised in that the active compound or granules thereof is/are coated with an amount of 3 to 10% of poly-D(-)-3-hydroxybutyric acid, based on the total weight.

4. Binder-free granules according to any one of Claims 1 to 3, characterised in that the molecular weight of the poly-D(-)-3-hydroxybutyric acid used is between 50,000 and 800,000.

**5.** Binder-free granules according to any one of Claims 1 to 4, characterised in that the molecular weight of the poly-D(-)-3-hydroxybutyric acid used is between 100,000 and 400,000.

**6.** Binder-free granules according to any one of Claims 1 to 5, characterised in that the active compound is celiprolol hydrochloride.

**7.** Binder-free granules according to any one of Claims 1 to 6, characterised in that they have been prepared by fluidised bed granulation.

**8.** Process for the preparation of binder-free granules for oral or parenteral administration with delayed release of the active compound, consisting of poly-D(-)-3-hydroxybutyric acid and a pharmaceutical active compound, characterised in that the pharmaceutical active compound or granules thereof is/are sprayed with a solution of poly-D(-)-3-hydroxybutyric acid in a building-up granulation process and the solvent is evaporated off, the active substance or its granules being coated with an amount of from 1 to 20% by weight of poly-D(-)-3-hydroxybutyric acid, based on the total weight.

**9.** Process according to Claim 8, characterised in that the building-up granulation process is a fluidised bed granulation process.

**10.** Process according to any one of Claims 7 to 9, characterised in that the binder-free granules are pressed to a drug form.

**Claims for the following Contracting States : ES, GR**

**1.** Process for the preparation of binder-free granules for oral parenteral administration with delayed release of the active compound consisting of poly-D(-)-3-hydroxybutyric acid and a pharmaceutical active compound, characterised in that the pharmaceutical active compound or granules thereof is/are sprayed with a solution of poly-D(-)-3-hydroxybutyric acid in a building-up granulation process and the solvent is evaporated off, the active substance or its granules being coated with an amount of 1 to 20% by weight of poly-D(-)-3-hydroxybutyric acid, based on the total weight.

**2.** Process according to Claim 1, the active compound or granules thereof being coated with an amount of 1 to 15% of poly-D(-)-3-hydroxybutyric acid, based on the total weight.

**3.** Process according to Claim 2, characterised in that the active compound or granules thereof is/are coated with an amount of 3 to 10% of poly-D(-)-3-hydroxybutyric acid, based on the total weight.

**4.** Process according to any one of Claims 1 to 3, characterised in that the molecular weight of the poly-D(-)-3-hydroxybutyric acid used is between 50,000 and 800,000.

**5.** Process according to any one of Claims 1 to 4, characterised in that the molecular weight of the poly-D(-)-3-hydroxybutyric acid used is between 100,000 and 400,000.

**6.** Process according to any one of Claims 1 to 5, characterised in that the active compound is celiprolol hydrochloride.

**7.** Process according to any one of Claims 1 to 6, characterised in that the building-up granulation process is a fluidised bed granulation process.

**8.** Process according to any one of Claims 1 to 7, characterised in that the binder-free granules are pressed to a drug form.

**Revendications**
**Revendications pour les Etats contractants suivants : AT, BE, CH, DE, FR, GB, IT, LI, LU, NL, SE**

**1.** Granule exempt de liant destiné à des applications orales ou parentérales à libération prolongée constituée d'un principe Actif pharmaceutique ou son granule et d'acide poly-D(-)-3-hydroxybutyrique, caractérisé en ce que le principe actif ou son granule est enrobé avec une quantité de 1 à 20% en masse d'acide poly-D(-)-3-hydroxybutyrique calculée sur la masse totale.

2. Granule exempt de liant selon la revendication 1, caractérisé en ce que le principe actif ou son granule est enrobé avec une quantité de 1 à 15% en masse d'acide poly-D(-)-3-hydroxybutyrique calculée sur la masse totale.

3. Granule exempt de liant selon la revendication 2, caractérisé en ce que le principe actif ou son granule est enrobé avec une quantité de 3 à 10% en masse d'acide poly-D(-)-3-hydroxybutyrique, calculée sur la masse totale.

4. Granule exempt de liant selon l'une des revendications 1 à 3, caractérisé en ce que la masse molaire de l'acide poly-D-(-)-3-hydroxybutyrique utilisé est comprise entre 50 000 et 800 000.

5. Granule exempt de liant selon l'une des revendications 1 à 4, caractérisé en ce que la masse molaire de l'acide poly-D-(-)-3-hydroxybutyrique utilisé est comprise entre 100 000 et 400 000.

6. Granule exempt de liant selon l'une des revendications 1 à 5, caractérisé en ce que le principe actif est constitué par le chlorydrate de céliprolole.

7. Granule exempt de liant selon l'une des revendications 1 à 6, caractérisé en ce qu'il est obtenu par granulation en lit fluidisé.

8. Procédé pour préparer un granule exempt de liant pour des applications orales ou parentérales à libération prolongée du principe actif comprenant l'acide poly-D-(-)-3-hydroxybutyrique et un principe actif pharmaceutique, caractérisé en ce qu'on pulvérise le principe actif ou son granule avec une solution d'acide poly-D-(-)-3-hydroxybutyrique suivant un procédé de granulation et on vaporise le solvant, le principe actif ou son granule étant enrobé avec une quantité de 1 à 20% en masse d'acide poly-D(-)-3-hydroxybutyrique, calculée sur la masse totale.

9. Procédé selon la revendication 8, caractérisé en ce que le procédé de préparation par granulation est un procédé de granulation en lit fluidisé.

10. Procédé selon l'une des revendications 7 à 9, caractérisé en ce que le granule exempt de liant est comprimé en une forme médicamenteuse.

**Revendications pour les Etats contractants suivants : ES, GR**

1. Procédé pour préparer un granule exempt de liant pour des applications orales ou parentérales à libération prolongée du principe actif comprenant l'acide poly-D-(-)-3-hydroxybutyrique et un principe actif pharmaceutique, caractérisé en ce qu'on pulvérise le principe actif ou son granule avec une solution d'acide poly-D-(-)-3-hydroxybutyrique suivant un procédé de granulation et on vaporise le solvant, le principe actif ou son granule étant enrobé avec une quantité de 1 à 20 % en masse d'acide poly-D-(-)-3-hydroxybutyrique, calculée sur la masse totale.

2. Procédé selon la revendication 1, caractérisé en ce que le principe actif ou son granule est enrobé avec une quantité de 1 à 15 % en masse d'acide poly-D-(-)-3-hydroxybutyrique, calculée sur la masse totale.

3. Procédé selon la revendication 2, caractérisé en ce que le principe actif ou son granule est enrobé avec une quantité de 3 à 10 % en masse d'acide poly-D-(-)-3-hydroxybutyrique, calculée sur la masse totale.

4. Procédé selon l'une quelconque des revendications 1 à 3, caractérisé en ce que la masse molaire de l'acide poly-D-(-)-3-hydroxybutyrique utilisé est comprise entre 50 000 et 800 000.

5. Procédé selon l'une quelconque des revendicotions 1 à 4, caractérisé en ce que la masse molaire de l'acide poly-D-(-)-3-hydroxybutyrique utilisé est comprise entre 100 000 et 400 000.

6. Procédé selon l'une quelconque des revendications 1 à 5, caractérisé en ce que le principe actif est constitué par le chlorhydrate de céliprolole.

7. Procédé selon l'une quelconque des revendications 1 à 6, caractérisé en ce qu'il est obtenu par granulation en lit fluidisé.

8. Procédé selon l'une quelconque des revendications 1 à 7, caractérisé en ce que le granule exempt de liant est comprimé en une forme médicamenteuse.